# EUROPEAN PATENT SPECIFICATION

(11) **EP 0 448 180 B1**
(45) Date of publication and mention of the grant of the patent: **20.05.1998**
(21) Application number: 91200684.8
(22) Date of filing: 25.03.1991
(51) Int. Cl.: C12P 1/00, C12P 35/00, C12P 37/00, C12N 15/00, C12N 15/52, C12N 15/81, A61K 39/395

(54) **A method of modulating the production of secondary metabolites**
Verfahren zur Modulierung der Produktion von sekundären Metaboliten
Méthode pour moduler la production des métabolites secondaires

(30) Priority: 23.03.1990 EP 90200695
(43) Date of publication of application: 25.09.1991
(73) Proprietor: GIST-BROCADES N.V., NL-2600 MA Delft (NL)
(72) Inventor: Van der Voort, Lucia Helena Marie, NL-2611 NS Delft (NL); Bovenberg, Roelof Ary Lans, NL-3062 ZD Rotterdam (NL); Muller, Walraven Henry, NL-3317 JK Dordrecht (NL); Verkley, Adrianus Johannes, NL-3438 QL Nieuwegein (NL)
(74) Representative: Matulewicz, Emil Rudolf Antonius, Dr.

(56) References cited:
- EP-A- 0 354 624
- J. CELL BIOLOGY vol. 108, no. 5, May 1989, ROCKEFELLER UNIV. PRESS, N.Y. , US; pages 1657 - 1664; S.J. GOULD ET AL.: 'A conserved tripeptide sorts proteins to peroxisomes'
- BIOCHIMICA ET BIOPHYSICA ACTA vol. 1008, 1989, ELSEVIER , AMSTERDAM, NL; pages 1 - 13; P. BORST: 'Peroxisome biogenesis revisited'
- GENE vol. 83, 1989, ELSEVIER PUBLISHERS, N.Y., U.S.; pages 291 - 300; J.L. BARREDO et al.: 'Cloning and characterization of the acyl-coenzyme A: 6-aminopenicillanic-acid-acyltransferase gene of Penicillium chrysogenum'

## Description

### INTRODUCTION

### Technical Field

The present invention relates to a method of modulating the production of secondary metabolites as exemplified by penicillins and cephalosporins.

### Background

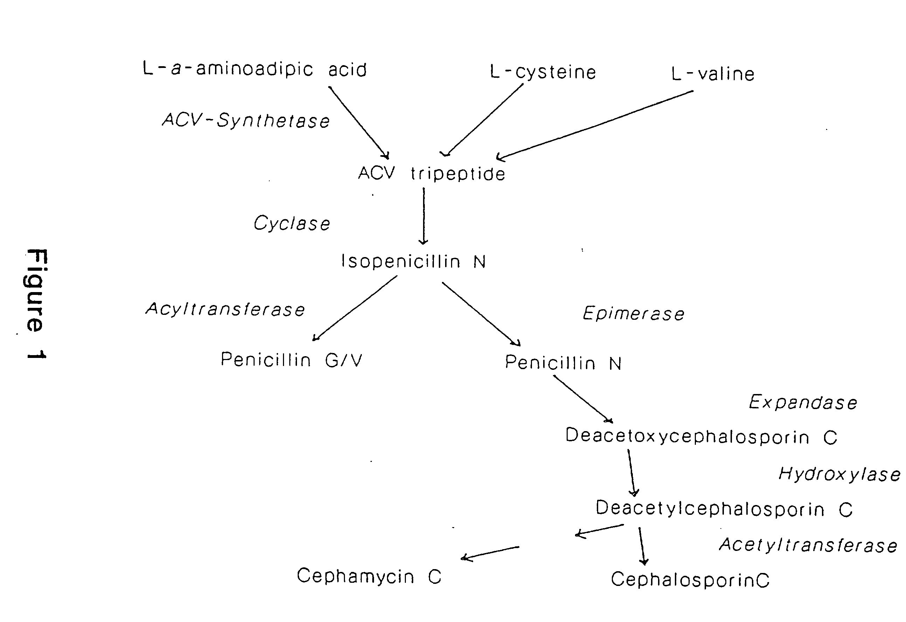

β-Lactam antibiotics are a large family of secondary metabolites produced in nature by microorganisms: Secondary metabolites are metabolites which have no known essential metabolic function in the producer organism. The most important classes of the β-lactam antibiotics both clinically and economically are the penicillins (penam) and cephalosporins (cephem). Their biosynthesis occurs via a complex pathway of enzymatic steps; the unravelling of this pathway has been the subject of many studies during the last few decades. The first two steps in the biosynthetic pathways of the penam and cephem classes of β-lactam antibiotics are identical. Thereafter the biosynthetic pathways to the penicillins and cephalosporins diverge (Figure 1).

The first step in the biosynthesis of the penicillin, cephalosporin and cephamycin antibiotics is the condensation of the L-isomers of three amino acids, L-α-amino adipic acid (A), L-cysteine (C) and L-valine (V) into a tripeptide, δ-(L-α-aminoadipyl)-L-cysteinyl-D-valine or ACV. In the second step, the tripeptide ACV is oxidatively cyclized by the action of the Isopenicillin N synthase (hereinafter referred to as IPNS) or cyclase. The product of this reaction is Isopenicillin N; this compound contains the typical β-lactam and thiazolidine ring structures and possesses antibacterial activity. From Isopenicillin N the penicillins G or V are formed by exchange of the hydrophilic α-AAA side chain by a hydrophobic side chain. The side chains commonly used in industrial processes are either phenylacetic acid (PA), yielding penicillin G, or phenoxyacetic acid (POA), yielding penicillin V. In vitro the side-chain precursor has to be activated before transacylation occurs; it has been suggested that in vivo this activation is catalyzed by a PAA Coenzyme A ligase. The exchange reaction is catalyzed by the enzyme acyltransferase (hereinafter referred to as AT).

From Isopenicillin N the route to cephalosporin and the cephamycins proceeds via racemisation of the α-AAA side chain, forming penicillin N. This reaction is catalyzed by an enzyme named epimerase or racemase. The five-membered ring in penicillin N is expanded into a six-membered ring by the action of the enzyme deacetoxycephalosporin C synthase or expandase. The fungal enzyme has been shown also to catalyze the next reaction in the pathway, the hydroxylation of the methyl group at the 3'-position of the six-membered ring, forming deacetylcephalosporin C. In Streptomycetes this latter enzyme activity is encoded by a separate gene. From deacetylcephalosporin C, Cephalosporin C is formed by acetylation of the 3'-position. The cephamycins are formed from deacetylcephalosporin C by several enzymatic steps.

Most of the enzymes involved in the biosynthesis of β-lactams have now been characterized. The genes of ACVS, IPNS and AT have been cloned and can be used in strain improvement programs. However, little is known about the other aspects of penicillin biosynthesis such as the localization of the enzymes and the transport of intermediates and products of the pathway. In this patent application we describe the location of AT in mycelial cells. Based on the localization of enzymes, methods are presented which allow for the modulation of the production of secondary metabolites such as penam and cephem compounds.

### Relevant Literature

Secondary metabolism relates to the production of metabolites which have no essential metabolic function in the producer organism. For further definition and review see Industrial Aspects of Biochemistry Vol. 30, part 1, J.D. Bu'lock (1974), p. 335, ed. B. Spencer, North Holland/ American Elsevier, Holland; and L.C. Vining, Biotechnology, a comprehensive treatise in eight volumes, Vol. 4, p. 20-38, H.J. Rehm and G. Reed (eds), 1986, VCH, Weinheim, Germany.

References on the biosynthetic pathways to β-lactams can be found in Ingolia and Queener, Med. Res. Rev. (1989), 9:245-264.

The biosynthesis of most of the cellular proteins starts in the cytoplasm. Based on the presence of specific amino acid sequences they are transported to different compartments of the cell. The major pathways of protein traffic are described in Molecular Biology of the Cell, Alberts et al. (eds), 1989, Garland Publ. Inc., New York).

The enzymes ACVS, IPNS and AT have been purified. ACVS is a large multifunctional enzyme with an apparent molecular weight of more than 250 kilodaltons (kDa) (Van Liempt et al., J. Biol. Chem. (1989), 264:3680-3684). The apparent molecular weight of IPNS is 39 kDa (Ramos et al., Antimicrobial Agents and Chemother. (1985) 27:380-387). Purified enzyme preparations of AT contain two polypeptides with apparent molecular weights of 29 and 10 kDa (see EP-A-336446). The genes of ACVS, IPNS and AT have been cloned (Carr et al., Gene (1986) 48:41-75; EP-A-320272, EP-A-354624, Barredo et al., Gene (1989) 83:291-300). The clustering of the three genes has been described in EP-A-320727 and in EP-A-357119. The PAA Coenzyme A ligase has been described by Brunner and Röhr, Methods in Enzymology (1975), 53:476-481.

References relating to the cellular localization of enzymes involved in penicillin biosynthesis are as follows. Kurylowicz et al. have suggested that the production of penicillin takes place in the vesicles of the Golgi apparatus (Kurylowicz et al., Zbl. Bakt. II Abt. (1979), 134: 706-720; Atlas of Ultrastructure of Penicillium chrysogenum in Course of Biosynthesis of Penicillin, Kurylowicz et al. (eds), 1980, Chemia Publ. Office, Warsaw; Kurylowicz et al., Archivum Immunologiae et Therapeae Experimentalis (1987), 35:699-724). Kurylowicz et al. (1987), supra, proposed that penicillin is produced in 40-200 nm Golgi vesicles, after which penicillin is transported to the plasma membrane by 40 nm vesicles. These vesicles subsequently fuse with the plasma membrane and release their contents in the fermentation broth. This hypothesis was based on the electron microscopical observation that penicillin G is accumulated inside substructures composed of small vesicles or in single vesicles in the mycelium of a high-yielding strain. Furthermore Kurylowicz et al. (1987), supra, describe cell fractionation experiments, from which they claim that ACVS, IPNS, PAA-CoA ligase and AT activities are present in Golgi vesicles ranging from 40-200 nm in size. After immobilization of vesicles of 40-200 nm, cell-free biosynthesis of penicillin was demonstrated. However, the evidence and interpretation for localizing penicillin biosynthetic enzymes in vesicles of the Golgi apparatus is not very convincing since, (1) the classical thin sectioning method which they used to prepare samples for electron microscopy may easily introduce artefacts in ultrastructure and localization (Cryotechniques in Biological Electron Microscopy, Knoll et al., p. 258-271, R.A. Steinbrecht and K. Zierold (eds), 1987, Springer Verlag, Berlin; and Plattner and Zingsheim, Subcellular Biochemistry, Vol. 9, p. 1-236, D.B. Roodyn (ed), 1983, Plenum Press, New York), and (2) fractions obtained in cell fractionation experiments were not characterized by marker enzymes and electron microscopy.

The localization of penicillin biosynthetic enzymes has been studied by a few other groups of investigators. ACVS has been reported to be exclusively associated with a particulate fraction of the crude homogenate of mycelium (Fawcett and Abraham, Methods Enzymol. (1975), 43:471-473; Fawcett and Abraham, Biosynthesis (1976), 4:248-265), which indicates that it might be associated with cellular membranes. Controversial data exist on the localization of IPNS in Cephalosporium. Abraham et al., (Recent Advances in the Chemistry of β-Lactam Antibiotics, p. 125-134, G.I. Gregory (ed), 1981, Royal Society of Chemistry, London) describe it as a cytosolic enzyme since it does not precipitate on high speed centrifugation. However, Sawada et al., Antimicrobial Agents Chemother. (1980), 18:465-470) showed that IPNS activity was stimulated by sonication and by addition of a detergent, which indicates that the enzyme is enclosed in vesicles. Luengo et al. have stated (in a paper presented at the 2nd Eur. Congr. Biotechnol., Eastbourne, England, 1981) that the side-chain exchange reaction is confined to the periplasmic space. This has not been confirmed until now. The localization of cephalosporin biosynthetic enzymes has not been described with any detail.

For a review of the ultrastructure of fungal mycelia see The Filamentous Fungi, Volumes 1-4, Smith and Berry (eds), 1979, Academic Press, London). Other references relating to compartments within fungal mycelium include the following: Markham and Collinge, FEMS Microbiology Reviews (1987), 46:1-11; Head et al., Experimental Mycology, 1989, 13:203-211; Smith and Berry, supra; Gooday in Fungal Differentiation, a Contemporary Synthesis, Mycology series, p. 315-365 (1983), J.E. Smith and P.A. Lenke (eds), Marcel Dekker, New York).

For review articles on vacuoles, see Molecular Biology of the Cell (supra) and Kurylowicz et al. (1980, supra). Organelles which can be found in all types of eukaryotic cells, including fungi, are nuclei, mitochondria, endoplasmic reticulum, the Golgi apparatus and microbodies; for review, see Molecular Biology of the Cell (supra) and Smith and Berry (supra). Microbodies are organelles with a single membrane. Examples of enzymes contained in microbodies include those involved in β-oxidation of fatty acids (Borst, Biochimica et Biophysica Acta, 1989, 1008:1-13). Microbodies may contain catalase. In this case they are often referred to as peroxisomes. Microbodies may also contain enzymes of the glyoxylate cycle. Then they are often referred to as glyoxisomes. Microbodies can adapt to changes in metabolic conditions by changes in enzyme content. In fungal cells their size varies between 0.2 and 2.6 µm (Maxwell et al., Planta (1975), 124:109-123). References to the targeting signals of proteins to microbodies can be found in Gould et al., J. Cell Biol., (1989) 108:1657-1664.

### SUMMARY OF THE INVENTION

Methods, and compositions for use therein, are provided for modulating production of secondary metabolites. The methods include the steps of
modulating the cellular localization of at least one protein, optionally derived from another microorganism, directly or indirectly involved in the production of said secondary metabolites by adding, deleting or altering one or more DNA sequences encoding one or more targeting signals in the gene(s) of one or more of said proteins.
The subject invention finds use particularly in the modulation of the production of penicillins and cephalosporins.

### BRIEF DESCRIPTION OF THE FIGURES

Figure 1 shows schematically the biosynthetic pathway to β-lactam antibiotics.

Figure 2 shows detection of AT by immunogold labelling in an organelle in the cytoplasm of Wisconsin 54-1255 cells.

Figure 3 shows (a) young hyphal cell with gold-labelled microbodies (arrow heads); higher magnification of the microbodies is shown to the right; (b) old hyphal cell exhibiting a large vacuole, a nucleus and a gold-labelled microbody (arrow head); higher magnification of the microbody is shown below.

Figure 4 shows immunoblots of cell fractions, stained with antisera against ACVS, IPNS and AT; molecular weight markers are indicated to the left; the bands corresponding to ACVS, IPNS and AT are indicated with an arrow.

Figure 5 shows a schematic representation of pMA-AT.

Figure 6 shows a schematic representation of pMC-AT.

Figure 7 shows a schematic representation of the mutations introduced in the penDE gene. The nucleotide sequences of Oligonucleotides S (Oli S) and D (Oli D) and a partial nucleotide sequence of the penDE is shown as well as the amino acid sequence (in one-letter code) deduced from the DNA sequences.

### BRIEF DESCRIPTION OF THE SPECIFIC EMBODIMENTS

In accordance with the subject invention, methods and compositions are provided which allow for modulating the production of secondary metabolites. The methods are based on the knowledge of the localization of proteins involved in the production of secondary metabolites. The term modulating within the context of this application intends all conceivable manners of alteration, viz. to enhance, to create, to diminish or to destroy. Localization intends both a specific location as well as a process of direction to a specific location within the cell.

Methods to modulate the production of secondary metabolites include modulating the cellular localization of one or more proteins which are directly or indirectly involved in the production of a particular secondary metabolite, by adding, deleting or altering the DNA sequence encoding (a) targeting signal(s) of one or more of said proteins. A targeting signal is defined herein as amino acid sequences in a polypeptide which cause the polypeptide to be localized in one of the cell organelles. This definition also includes retention signals, for instance amino acid sequences which cause the protein to be localized in the endoplasmic reticulum.

The proteins may be directly or indirectly involved in the production of said secondary metabolite. Proteins directly involved are enzymes which are essential in the biosynthetic routes to said secondary metabolite, such as for instance the penicillin biosynthetic enzymes. Proteins which are indirectly involved in the production of secondary metabolites are those proteins of which expression and/or activity has an influence on the production of said secondary metabolites. Such proteins are for instance involved in maintaining the conditions which are proper to the production of said secondary metabolite, such as the pH and the availability of ATP, or are involved in transport of proteins, or substrates and products belonging to the biosynthetic pathway.

The advantages associated with these methods include the following. The improvement of the production of secondary metabolites by altering the biosynthetic pathway in such a way that intermediates thereof do not have to be transported from one cellular location to another. This is obtained by co-localization of two or more enzymes which are involved in the production of a secondary metabolite, in the cytoplasm or in a particular organelle. A further advantage is the creation of a pathway leading to a particular secondary metabolite, which pathway is by nature not present in a particular microorganism. This can be achieved by providing one or more proteins directly or indirectly involved in the biosynthesis of said secondary metabolite, which proteins are by nature not present in said organism, with a targeting signal for organelles containing substrate to one of the enzymes involved in the production of secondary metabolite, and cloning these proteins into said microorganism. The production of a secondary metabolite can also be improved or facilitated by the use of an organism which is by nature not able to produce this metabolite. This can be accomplished by providing one or more proteins directly or indirectly involved in the production of said secondary metabolite with a targeting signal for a particular organelle and cloning these proteins into said microorganism. Yet another advantage is the use of genes encoding enzymes which do not have targeting signals, such as for instance bacterial enzymes for strain improvement of eukaryotic microorganisms involved in the production of a particular secondary metabolite, by providing these enzymes with a targeting signal for an organelle which is involved in the production of said secondary metabolite and cloning the altered gene into the microorganism. Further advantages are the improvement of production of a secondary metabolite by providing proteins directly or indirectly involved in the biosynthesis thereof with an altered targeting signal, which results in an improved or a decreased amount of protein in the organelle(s) involved in production. The improvement of production of a secondary metabolite can also be obtained by removal or addition of a targeting signal from or to one or more proteins directly or indirectly involved in the production of said secondary metabolite, to remove or add the protein from or to an organelle involved in the production of said secondary metabolite. Secondary metabolites include metabolites such as those derived from saccharides, for instance kojic acid, those derived from amino acids, such as β-lactam compounds and alkaloids, those derived from acyl precursors, such as carotenoids or polyketides, those with a relationship to nucleic acids and those of mixed biogenesis, such as the prodigiosine family of pigments and fluoro acetic acids.

To determine the cellular localization of proteins related to the production of a secondary metabolite of interest, the ultrastructure of a cell which synthesizes the secondary metabolite is visualized at the microscopical level, preferably the electron microscopical level. The technique used for preparing samples should provide optimal preservation of native cell structures for thin sectioning, such as for instance is obtained by fast-freezing and freeze-substitution; for review see Immunogold Labelling in Cell Biology, A.J. Verkley and J.L.M. Leunissen (eds) CRC Press, Boca Raton, Florida (1989). The proteins are localized for instance by cytochemical methods, but preferably by immunocytochemical methods, for instance the protein A/gold labelling method. A method which may confirm the cellular localization of proteins is cell fractionation. For a description of these methods, see Cell Biology, a Molecular Approach, R.D. Dyson, 1978; and Biological Mem branes, a Practical Approach, Findlay and Evans (eds), 1987, IRL Press, Oxford. Organelles can be partially separated by cell fractionation experiments. The cells are gently broken and the homogenate is subjected to centrifugation steps of increasing velocity. The organelles have different sedimentation rates. In typical experiments with animal cells, nuclei and unbroken cells sediment at 6.10² g x 10 min, mitochondria, microbodies and lysosomes sediment at 15.10³ g x 5 min, and endoplasmic reticulum sediments at 100.10³ g x 60 min. The remaining supernatant is the soluble portion of the cytoplasm, the cytosol (Cell Biology, A Molecular Approach, supra). The successful fractionation of organelles is very dependent on a careful breaking of cells. Especially the integrity of microbodies is easily affected by lysis of the cells (Kionka et al., J. Bacteriology (1985), 161:153-157). The presence of the proteins of interest in cell fractions can be determined, for instance, if the protein is an enzyme by an assay of the activity, or by immunoblotting. The cellular localization of enzymes related to the production of secondary metabolites of interest is also inferred from alignment of the nucleotide sequences of the gene encoding the enzyme to consensus sequences of targeting signals.

The host organism may be selectively manipulated by techniques which produce changes in the localization of proteins to or from an organelle. Techniques will be described in terms of changing a cellular location to or from a microbody, but any organelle may be substituted. The localization of the proteins, directly or indirectly involved in the production of secondary metabolites, can be altered either from the natural location in the microbody to other locations in the cell, or from the natural location of other parts of the cell than microbodies to the microbody by adding, deleting or altering the DNA sequence encoding the microbody targeting signal of said protein.

Targeting signals for microbodies are for instance the conserved tripeptide sequence described by Gould (supra). The methods also apply to other amino acid sequences which are involved in the targeting of proteins to microbodies. Sequences involved in targeting can be identified, for instance, by the methods described by Gould (supra), or by comparing amino acid sequences of genes with consensus sequences for targeting signals.

Microbody targeting.sequences can be altered, removed from or added to proteins by site-directed mutagenesis of the gene encoding the protein of interest. Techniques and strategies for site-directed mutagenesis are abundantly available, well known in the art and well documented (Smith, Annual Review of Genetics (1985), 19:423-462; Molecular Cloning: a Laboratory Manual, Maniatis et al. (1989), Cold Spring Harbor Laboratory Press, Cold Spring Harbor; Innes, M.A., Gelfand, D.H., Sninsky, J.J. and White, T.J. (eds), PCR Protocols: a Guide to Methods and Applications (1990), Academic Press, San Diego). Two major approaches are applied preferably. Basically, one approach employs the isolation of single-stranded (ss) DNA from a plasmid containing the gene of interest, annealing of this ss DNA with a synthetic oligonucleotide containing the mutation and in vitro synthesis of (part of) the complementary strand. The duplex DNA is transformed to E. coli and double-stranded (ds) mutant plasmid molecules are selected, typically by hybridization with the mutant oligonucleotide. Another major approach employs the Polymerase Chain Reaction (PCR). In this approach the desired mutations are also designed in synthetic oligonucleotides, but in contrast to the first approach it is not necessary to clone the gene of interest first.

The signal may be provided at the C-terminal part of the polypeptide or in the internal regions. Manipulations include the removal of (a) targeting signal(s) from a protein for organelles other than microbodies and the addition of (a) targeting signal(s) for microbodies; the addition of a microbody targeting signal to a protein which has no other targeting signals; the removal, alteration, or destruction of a microbody targeting signal; or exchanging one microbody targeting signal for another.

Exemplary for the use of the above methods in the production of β-lactam compounds is the following.

The localization of AT was investigated by immuno-electron microscopy. AT was present in single membrane vesicles with a diameter ranging from 200-800 nm. Vesicles containing AT were present in young cells and in older compartments of the hyphae. Based on morphology, size and distribution through the hyphae these vesicles can be classified as microbodies. In cell fractionation experiments AT activity was sedimented at 15.10³ g x 30 min together with enzyme activities of the mitochondria and the vacuole. Catalase activity has also been found in this fraction. IPNS activity is located in a fraction corresponding to cytosolic enzymes. These results were confirmed by immunoblotting experiments. Furthermore, it has been shown in these experiments that ACVS is located in yet another cellular fraction which sediments at 100.10³ g x 60 min. This demonstrates that one of the steps of penicillin biosynthesis is localized in microbodies. By comparing strains with high-yielding and low-yielding capacities for the production of penicillin the existence of a clear correlation between the number of microbodies containing AT and the amount of penicillin was shown. Furthermore, the number of microbodies containing AT in high-yielding strains was reduced in non-penicillin producing conditions. These examples indicate that modulation of the number and/or size of microbodies containing AT is an important means of improving penicillin production.

Our localization experiments further demonstrate that IPNS is not present in said microbodies. This implies that substrates and products of enzymes, involved in the production of β-lactam compounds, and located in microbodies, for instance substrates of the enzyme AT, such as isopenicillin N and PAA or activated PAA, and products, such as penicillin G and α-aminoadipic acid must be imported into said microbodies or exported from said microbodies. These import and export systems form part of the microbodies involved in penicillin production. They are also an integral part of the penicillin biosynthetic route and may thus be responsible for the rate-limiting step within the biosynthetic route. By manipulating the expression of these systems, for instance by introducing extra copies of the genes encoding for these systems, the efficiency of production of penicillin will be improved.

Exemplary for the ability to influence the production of β-lactam antibiotics by changing the localization of one or more proteins, involved in β-lactam biosynthesis, is the altering of the localization of AT. AT contains the C-terminal amino acid sequence ARL (A = alanine, R = arginine and L = leucine; ARL is one of the microbody targeting sequences mentioned by Gould (supra)). The ARL targeting signal was removed from the gene encoding AT, followed by cloning of the altered gene into P. chrysogenum. AT was expressed in the transformants, and cell-free extracts prepared from the transformants were active in the AT activity assay. The transformants did not produce penicillin in detectable amounts, which proves that it is possible to influence the production of β-lactam antibiotics by changing the localization of AT. In this experimental setting the enzyme involved in the activation of the side-chain precursor was not re-located, which may explain why co-localization of IPNS and AT did not result in improved production.

A further example for the application of the present invention is the addition of a targeting signal for a specific organelle to β-lactam biosynthetic enzymes from prokaryotic organisms. These enzymes may have a better stability or a higher activity or a broader substrate range than the enzymes with a similar activity from eukaryotic organisms. Prokaryotic genes generally do not encode targeting signals for eukaryotic organelles. By adding a targeting signal to the prokaryotic enzyme, this enzyme can be directed to a desired location in the cell. The enzymes may be obtained from prokaryotic microorganisms, preferably from Streptomyces, Nocardia and Flavobacterium species.

Yet another example is the production of β-lactam compounds which are not by nature produced in the particular organism, but which will be produced after introduction of the genes encoding for the proteins involved in the biosynthesis of said β-lactam compounds. For instance the cephalosporin biosynthetic genes are not present in Penicillium strains. Production of cephalosporins (such as cephalosporin C, its deacetyl or deacetoxy derivatives, 7-aminocephalosporanic acid, its deacetyl or deacetoxy derivatives, cephamycins) in Penicillium might be advantageous, for instance when strains are used which produce high amounts of isopenicillin N or when strains are used with improved growth or down stream processing characteristics. Most of the isopenicillin N which is formed by IPNS is transported to said microbodies. Epimerase, which is an enzyme involved in the biosynthesis of cephalosporin and said intermediates, has isopenicillin N as a substrate. By preventing the expression of AT in P. chrysogenum, for instance by gene disruption of the penDE gene, by the application of anti-sense technology, or by the use of mutants which do not express AT, and by localizing epimerase and preferably subsequent cephalosporin biosynthetic enzymes like expandase and hydroxylase e.g. from Acremonium chrysogenum or from Streptomyces strains, in said microbodies, the efficiency of the production of cephalosporin or said intermediates in P. chrysogenum will be improved.

According to this invention also the genes encoding the proteins wherein the DNA sequence has been altered as described above and DNA constructs comprising said genes have been provided for.

Preferably, the production of secondary metabolites according to the above-indicated method of modulating the cellular localization of one or more of the proteins, is carried out in eukaryotic microorganisms, more preferably in Penicillium chrysogenum, Acremonium chrysogenum or Aspergillus nidulans. The production can also very well be carried out in a microorganism which is by nature not able to produce said secondary metabolites, for instance the production of penicillins by co-localization of AT, IPNS and ACVS in microbodies of said microorganism, such as yeast.

Furthermore, according to the invention a eukaryotic microorganism, preferably P. chrysogenum, A. chrysogenum or A. nidulans, wherein the cellular localization of one or more of the proteins has been modulated by any of the above-indicated methods, is provided. These proteins may originate from eukaryotic organisms such as Penicillium, Acremonium and Aspergillus species or from prokaryotic organisms such as Streptomyces and Flavobacterium species.

The following examples further illustrate the invention.

### EXPERIMENTAL

### Microbiological Deposits

The mutant strains npe6 and npe10, both obtained from Wisconsin 54-1255, have been deposited at the Centraal Bureau voor Schimmelcultures, Oosterstraat 1, 3742 SK Baarn, The Netherlands. Strain npe10 has been deposited on March 13, 1990 under accession No. CBS 143.90. Strain npe6 has been deposited on February 19, 1991 under accession No. CBS 117.91.

### General Procedures

### A. Preparation and characterization of antibodies against AT. ACVS, and IPNS

### Antibodies against AT

Purification of acyltransferase was done essentially as described by Alvarez with minor modifications (Alvarez (1987), supra). Penicillium chrysogenum was cultured on production medium as described in EP-A-354624. Mycelium was disrupted by freezing in liquid nitrogen, followed by mortaring. The 0-45% ammonium sulphate fraction was used for further purification.

After purification, a preparation was obtained which contained two major polypeptides with apparent molecular weights of 29 kDa and 10 kDa. Both polypeptides are products of the AT gene as determined by comparing the N-terminal amino acid sequence of the bands with the nucleotide sequence of the gene by the same methods as described by Barredo et al. (1989), supra. To obtain pure polypeptides for immunization purposes, Sodium Dodecylsulphate Polyacrylamide Gel Electrophoresis (SDS-PAGE) was done as described by Laemmli, Nature (1970), 227:680). The 29 kDa band was excised from the SDS-PA gel and freeze-dried. The freeze-dried material was mortared and subsequently resuspended in 0.9% NaCl. The suspension was mixed with adjuvant system (Ribi Immunochem Research, Hamilton, Montana, U.S.A.). Rabbits were immunized subcutaneously and intramuscularly, boosters were given after 2, 4, and 6 weeks. For each rabbit, approximately 1 mg of purified polypeptide was used in the entire immunization procedure.

Blood was collected from the immunized rabbits and an IgG fraction was prepared from the serum by protein-G Sepharose affinity chromatography. To remove immunoglobulins which might adhere non-specifically to cell wall components, the IgG fraction was absorbed with intact mycelium. To this end, P. chrysogenum was grown for 4 days. The mycelium was washed three times with phosphate buffered saline. 0.4 g (wet weight) mycelium was resuspended in 20 ml of PBS. The IgG fraction was mixed with the washed mycelium (1:4) and incubated for 30 min at room temperature. The mycelium was then removed by low-speed centrifugation.

To investigate the specificity of the antibodies, the reactivity of the IgG fraction with cell-free extracts from Wisconsin 54-1255 (ATCC 28089) and npe10, a mutant thereof which lacks the entire penicillin biosynthetic cluster, was analyzed by immunoblotting. Cell-free extracts of npe10 were prepared as follows. Mycelium was frozen in liquid nitrogen, and the cells were broken by mortaring the frozen mycelium. The mortared material was resuspended in PED buffer (50 mM K₂HPO₄, 5 mM EDTA, 5 mM DTT and 1 mM PMSF (pH 7.5) at 4°C, 10 g powder/ 50 ml PED buffer). DNA was precipitated with protaminesulphate (0.4%). After 30 min at 4°C the precipitate was removed by centrifugation at 10.10³x g for 15 min. Immunoblotting was done as follows. Polypeptides were separated by SDS-PAGE in 13.3% (w/v) acrylamide separating gels (Mini PROTEAN II, BioRad). 20 µg of the cell-free extract was applied per lane. The polypeptides were electrophoretically transferred from the slab gel onto nitrocellulose sheets using a Multiphor II Nova blot apparatus (LKB) according to the procedure given by the manufacturers. Immunostaining was done with the Proto-Blot Western blot AP system (Promega) according to the procedure given by the manufacturers, IgG fractions were diluted in blocking buffer.

The IgG fraction reacts specifically with a 29 kDa band which is present in cell-free extracts obtained from the Wisconsin 54-1255 strain. No staining was observed with extracts prepared from the mutant strain npe10.

### Antibodies against ACVS and IPNS

Antiserum against ACVS was raised in rabbits against native ACVS which was purified from Aspergillus nidulans (Van Liempt et al., supra).

IPNS was purified as described by Ramos et al., (1985, supra). Analysis by SDS-PAGE revealed the presence of a major band with an apparent molecular mass of 39 kDa. This band was excised and used for immunization of rabbits according to the protocol described for AT.

IgG antibodies from the anti-ACVS and the anti-IPNS antisera were isolated as described above. To remove non-specific antibodies the IgG fractions were absorbed with extract from npe10 mycelium. 100 mg of freeze-dried and mortared mycelium was resuspended in 250 µl 50 mM Tris/HCl pH 7.5. This suspension was added to 500 µl IgG. After 1 hour at room temperature cell debris was removed by centrifugation for 15 min at 12.10³x g.

The specificity of the antisera was investigated by immunoblotting. To detect ACVS by immunoblotting, cell-free extracts were prepared as follows. Mycelium was frozen in liquid nitrogen and the cells were broken by mortaring the frozen mycelium. The mortared material was resuspended in buffer B (50 mM Tris/HCl, pH 7.5, 1 mM dithioerythritol, 0.1 mM EDTA, and 9% (w/v) glycerol). DNA was precipitated with Polymin B (0.1%), after 30 min at 4°C the precipitate was removed by centrifugation at 10.10³x g for 15 min. To the supernatant a saturated ammoniumsulphate solution was gradually added. The protein fraction precipitating between 0 and 45% saturation was taken up in buffer B. This material was precipitated by adding 1 volume of 20% w/v trichloroacetic acid. After 60 min at 4°C the precipitate was collected by centrifugation at 10.10³x g for 15 min. Since ACVS has an apparent molecular mass of 260 kDa, 5% (w/v) acrylamide gels were used.) Immunostaining was done as described above, 20 µg of cell-free extract per lane was applied.

The absorbed IgG fraction against IPNS reacted with a 39 kDa polypeptide in cell-free extracts from Wisconsin 54-1255, whereas no staining was observed with extracts obtained from npe10.

The absorbed IgG fraction against ACVS reacted with a 260 kDa polypeptide in cell-free extracts from Wisconsin 54-1255, whereas no staining was observed with extracts obtained from npe10.

### B. Electron-microscopical methods

### Classical thin sectioning

Submerged cultured Penicillium chrysogenum was washed with 0.1 M phosphate buffered saline (PBS, pH 7.4). The mycelium was chemically fixed with 2% paraformaldehyde, 0.5% glutaraldehyde, 0.1% DMSO and 2% tannic acid in 0.1 M PBS) for 48 hours at 4°C. After rinsing with PBS mycelial material was post-fixed with 1% aqueous OsO₄ solution for 1 hour at room temperature.

The chemically fixed mycelial material was rinsed with 50% acetone and transferred to a 70% acetone solution for 16 hours at 4°C. Block staining was done with a solution of uranyl acetate (1%) in 70% acetone at room temperature for 30 min. The material was dehydrated in a graded acetone series, and infiltrated with Araldit (Merck) according to the following schedule: 33%, 50% (1 hour each), 66% (16 hours), 75% (1 hour) and 100% Araldit (2 x 3 hours). Infiltrated material was put in Eppendorf tubes. The fluid embedding Araldit was immediately added to the mycelium. The embedding medium was hardened by incubation at 70°C for 48 hours. Thin sections (100 nm) were cut with a diamond knife on a Reichert-Jung ultramicrotome.

### Rapid freezing, freeze-substitution and low temperature embedding

Mycelium was prepared for rapid freezing in nitrogen pre-cooled liquid propane with the Reichert-Jung KF 80 apparatus (H. Sitte et al., 1985, In: The science of biological specimen preparation of microscopy and microanalysis, p. 103-118, M. Muller, R.P. Becker, A. Boyde, J.J. Wolosewich, S.A. Batho (eds), S.E.M. Inc, Chicago).

Freeze-substitution and low temperature embedding were performed in the Reichert-Jung CS auto substitution chamber at -90°C. While being transferred to the chamber care was taken to keep the frozen material at a temperature below -90°C. The frozen samples were placed in 0.5% uranyl acetate in pure methanol at -90°C. After 2 days the temperature was raised to -45°C at a rate of 5°C per hour. The samples were subsequently rinsed with anhydrous methanol and infiltrated with Lowicryl HM20 in three steps of increasing concentration, 50%, 66% and 100% Lowicryl HM20, each step lasting 2 hours. After 16 hours the specimens were transferred to an embedding mould, which was filled with Lowicryl HM20 at -45°C. Polymerization was for 48 hours at -45°C in the CS auto apparatus, followed by polymerization at room temperature for 48 hours in the CS auto apparatus by a UV light source (360 nm).

The Lowicryl HM20 blocks containing the specimens were removed from the embedding mould and cut perpendicularly or longitudinally with a Reichert-Jung ultramicrotome.

### Preparation and examination of thin sections

Thin sections prepared by classical thin sectioning and freeze-substitution at low temperature were mounted on 0.7% formvar-coated carbon-evaporated one hole grids, stained with 4% aqueous uranyl acetate for 15 min and lead citrate, or with 1% aqueous KMnO₄ for 10 min. Thin sections were examined in a Philips EM420 microscope at 80 kV.

### Freeze fracturing

The hyphae were freeze-fractured in a Balzers BAF300 apparatus. The samples were fractured at -105°C and were subsequently deposited with platinum/ carbon and carbon (Pt/C at an angle of 45°C and 90°C). After removal from the BAF300 and thawing the replicas were cleaned with 3% Cr/10% H₂SO₄ overnight and were subsequently washed in distilled water. The cleaned replicas were placed on an uncoated 400 mesh EM grid and examined in a Philips EM420 microscope at 80 kV.

### Immunogold labelling

Immunostaining of thin sections was performed as described by P.M.P. van Bergen en Henegouwen et al. (1986, Histochemistry 85:81-87). In short, thin sections were incubated with antibodies against AT. Preparation of IgG antibodies and absorption was done as described in General Procedures A, a 1:1000 dilution of the absorbed IgG fraction was used. After washing the thin sections were incubated with goat-anti-rabbit antibodies to which gold particles with a diameter of 10 nm were attached (Janssen Pharmaceuticals, Beerse, Belgium).

### Fast freezing versus chemical fixation

The ultrastructure of unfixed and chemically fixed mycelia was compared after freeze fracturing. Whereas the unfixed hyphal cells reveal smooth vacuolar and plasma membranes with homogeneously distributed intramembrane particles, chemically fixed cells show undulated vacuolar and plasma membranes with intramembrane particle segregation. When thin sections of chemically fixed hyphae were compared to thin sections of hyphae prepared by fast freezing and freeze substitution at low temperature, a conspicuous collapse of vacuoles was observed in thin sections prepared by the former method. This is consistent with the general consensus that fast freezing prevents structural alterations (Knoll, supra).

Furthermore, immunogold labelling with antibodies against AT was not possible with thin sections of chemically fixed hyphae, whereas positive staining was obtained with thin sections prepared by a combination of fast freezing and freeze-substitution. Very likely the antigenicity of polypeptides was destroyed by the former method as frequently observed by others in the field (Immunogold labelling in Cell Biology, supra).

### Example 1

### Localization of AT in specific organelles of Penicillium chrysogenum

P. chrysogenum strain Wisconsin 54-1255 and npe10 were grown in shake flasks on production medium (EP-A-354624). Thin sections were prepared by a combination of fast freezing and freeze-substitution as described in General Procedure B. Immunostaining was performed with antibodies against AT (see General Procedure A) as described in General Procedure B.

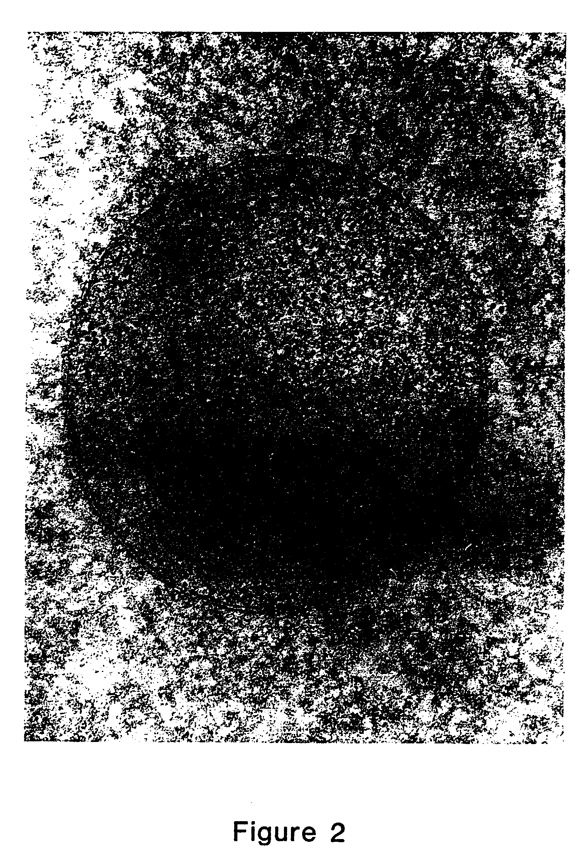

In thin sections prepared from npe10, a strain which lacks the penicillin biosynthetic gene cluster, no labelling could be detected. However in thin sections prepared from Wisconsin 54-1255 gold particles were predominantly detected in single membrane organelles with a diameter ranging from 200-800 nm. In contrast to vacuoles these organelles have an electron-dense content. The organelles were found throughout the cell. Sometimes the smaller organelles were found in association with the nucleus or with endoplasmic reticulum. A labelled organelle is shown in Figure 2.

### Example 2

### Presence of organelles containing AT throughout the hyphae

P. chrysogenum spores were grown between dissected dialysis tubing on agar plates containing the production medium. After 6 days the dialysis tubing containing colonies was removed from the plate and placed on a slide. The dialysis tubing was unfolded with a fine forceps. The colony and the supporting dialysis tubing were cut into squares with a razor blade, and the squares were placed on an agar plate for 15 min. The squares were subjected to fast freezing as described in General Procedure B.

Growth of P. chrysogenum colonies occurs radially and centrifugally in all directions. The younger cells are in the periphery and the older are in the centre of the colony. Specific colonial parts were investigated by longitudinal sectioning. The apical cell has many ribosomes, mitochondria and vesicles in the first third of the cell. Beside these organelles the second third of this cell contains also nuclei. The last third of the apical cell contains less nuclei and an abundance of small vacuoles. The size of these vacuoles increases towards the septum. The vacuoles in the subapical cells are larger than the vacuoles in the tip cell, but the number of vacuoles is less. In older cells very large vacuoles are found which occupy almost the total cell volume, resulting in a rim of cytoplasm between the vacuolar membrane and the plasma membrane.

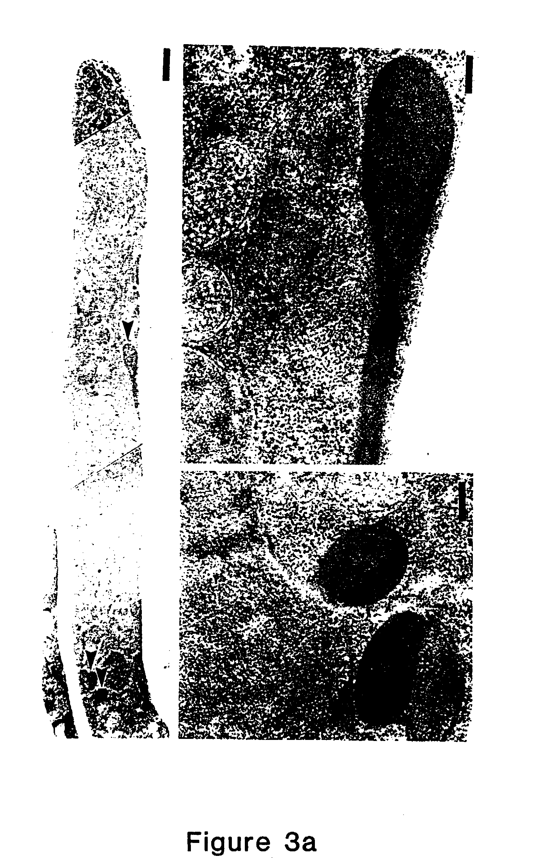

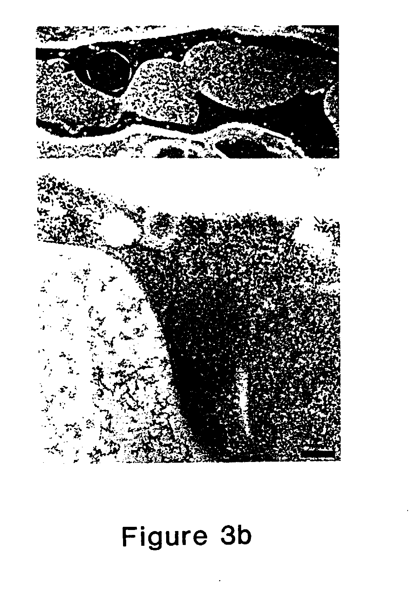

Immunostaining was performed on longitudinally cut thin sections of young cells as well as older cells as described in Example 1. Gold-labelled organelles were detected throughout the hyphae. The number of organelles detected per square surface of cell area did not differ conspicuously between younger and older cells, unless an old hyphal cell was totally occupied by a large vacuole. Examples of labelled organelles in younger and older cells are shown in Figure 3. Based on morphology, size and distribution through the hyphae these organelles are classified as microbodies.

### Example 3

### Cell fractionation of Penicillium chrysogenum

### Distribution of ACVS, IPNS and AT

P. chrysogenum strain P2 (ATCC 48271) was harvested 72 hours after inoculation. The mycelium was washed once with 0.9% NaCl. 20 gr (wet weight) was resuspended in 100 ml buffer A (2 gr/l citric acid, pH 6.4, 60 g KCl/l). Subsequently 500 mg Novozyme (Novo Biolabs, Bagsvaerd, Denmark) was added. The suspension was shaken for 4 hours at 25°C at 100 rpm. Protoplasts were washed three times with buffer B (0.1 M 2(N-morfolino)ethane sulphonic acid (MES), pH 7.5, containing 60 g KCl/l). The protoplasts were resuspended in buffer C (0.1 M MES, pH 7.5, 5 mM DTT, 1 mM EDTA, containing 25 g/l NaCl) and were further lysed by 5 passes with a Potter.

The suspension was cleared from debris by centrifugation at 1.1³x g for 15 min at 4°C. The supernatant (1KS) was fractionated in a pellet (14KP) and a supernatant (14KS) by centrifugation at 14.1³x g for 30 min at 4°C. Finally the 14KS supernatant was fractionated at 100.1³x g for 1 hour at 4°C, yielding the 100KS and 100KP fractions.

The polypeptides in the fractions, 1KS, 14KS, 14KP, 100KS and 100KP were separated by SDS-PAGE, and subsequently transferred to nitrocellulose as described in General Procedure A. Protein was determined as described by Peterson, Analytical Biochem. (1977), 83:346-356), 20 µg was applied per lane. Immunostaining was performed with the absorbed IgG fractions which were prepared as described in General Procedure A.

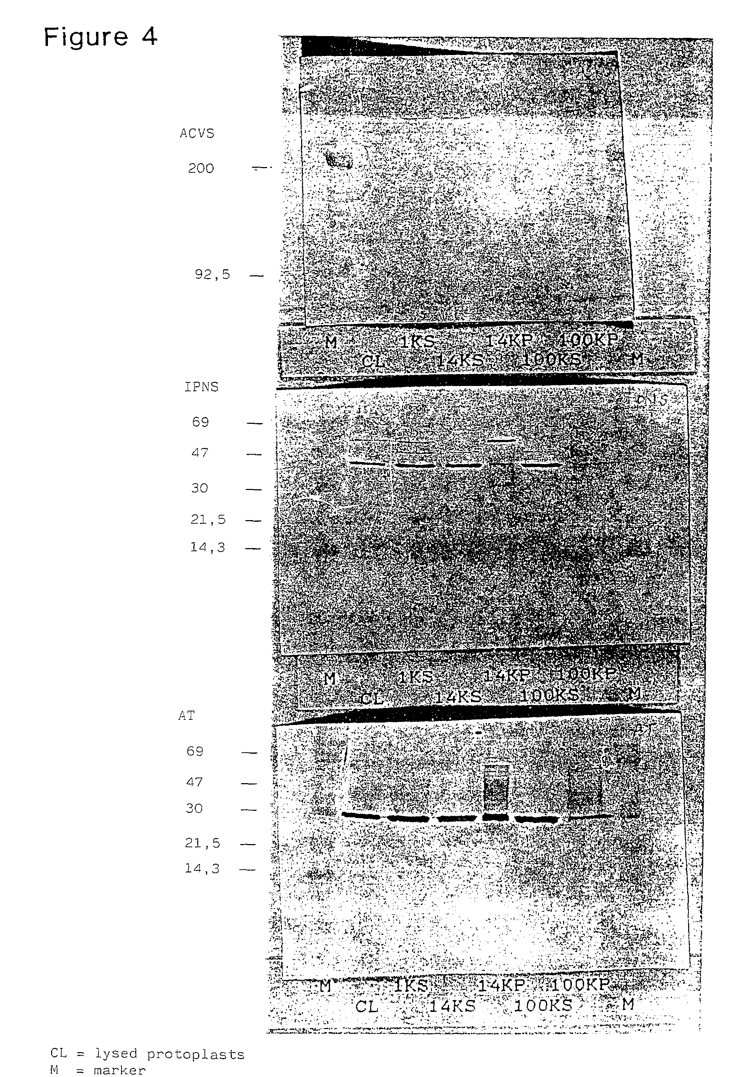

Immunostaining with antibodies against AT showed that AT was present in all fractions except 100KP. Immunostaining with antibodies against IPNS showed that IPNS was present in 1KS, 14KS and 100KS but not in 14KP and 100KP. Immunostaining with antibodies against ACVS showed that ACVS was present in 1KS, 14KS and 100KP. These results are shown in Figure 4.

From these experiments it is concluded that the three enzymes are present in different cellular fractions. IPNS behaves as a cytosolic enzyme. The enzyme ACVS is associated with the fraction corresponding to endoplasmic reticulum or other small membrane vesicles. AT is localized in organelles with a sedimentation rate corresponding to mitochondria, vacuoles and microbodies. The absence of AT in 100KP suggests that AT is not associated with small membrane vesicles. The presence of AT in all fractions except 100KP is explained by lysis of microbodies during cell fractionation. To confirm that AT is localized in microbodies in the 14KP fraction, the constituents of the 14KP fraction were visualized by electron microscopy. Ultrathin sections were prepared by a combination of fast freezing and freeze-substitution as described in General Procedure B. The pellet contained mitochondria and numerous vesicles of different sizes. Some of these vesicles contained electron dense material. By immunostaining of the 14KP with the IgG fraction against AT, it was shown that many of these vesicles contain AT. The size of these vesicles ranged from 200-800 nm. Vesicles containing AT were present in young cells and in older compartments of the hyphae. Based on morphology, size and distribution through the hyphae these vesicles can be classified as microbodies and not as Golgi vesicles.

### Example 4

### Cell fractionation experiments with Penicillium chrysogenum

### Distribution of marker enzyme activities and activities of IPNS and AT

Protoplasts of P. chrysogenum were fractionated as described in Example 3. The following marker enzymes were used: ATPase activity determined at pH 6.0 as a marker for the plasma membrane was determined as described by Navarette et al. (Navarette, Biochim. Biophys. Acta (1983), 728:403-408), 5'nucleotidase activity as a marker for the plasma membrane was determined as described by Onishi et al., Anal. Biochem. (1975), 69:261-267. Thiamine pyrophosphatase activity as a marker for the Golgi apparatus was determined as described by Morré (Meth. Enzymol. (1971), 22:138-139) except that the volume of the reaction mixture was 0.5 ml. Glucose-6-phosphatase activity as a marker for endoplasmic reticulum was determined as described by Morré (supra), except that the volume of the reaction mixture was 0.5 ml. Malate dehydrogenase activity as a marker for mitochondria was determined as described by Gross, Phytochem. (1977), 16:319-321. α-Mannosidase activity as a marker for vacuoles was determined as described by van der Wilden, Naturforschung (1973), 28:416-421). Catalase activity as a marker for peroxisomes was determined as described by Cohen et al., J. Anal. Biochem. (1970), 34:30-38.

AT activity was determined as described by Alvarez, supra, IPNS activity was determined as described by Ramos, supra.

The activity of the enzymes per mg of protein in the fractions 14KP, 100KS and 100KP was expressed as the fraction of the activity found per mg of protein in the 1KS fraction. Protein was determined as described by Peterson, supra.

The distribution of marker enzymes over the fractions 14KP, 100KS, and 100KP varied in different experiments, however, generally the 14KP fraction was enriched in the marker enzymes for malate dehydrogenase, α-mannosidase and catalase. The activity of thiamine pyrophosphatase was found in 14KP and as well as in 100KP. The highest AT activities were generally found in the 14KP fraction, however in some experiments the AT activity was found predominantly in the 100KS fraction. This may be related to the lysis of microbodies during lysis of the protoplasts. The highest activity of IPNS was always present in the 100KS fraction.

The distribution of marker enzymes in a typical experiment is exemplified; in Table 1.

**Table 1**

| | Fraction | | |
|---|---|---|---|
| Enzyme | 14KP | 100KP | 100KS |
| Cyclase | N.D. | N.D. | 0.46 |
| Acyltransferase | 1.13 | N.D. | 0.13 |
| ATPase pH 6.0 | 0.51 | 1.26 | 0.53 |
| 5'Nucleotidase | 0.78 | 0.47 | 1.10 |
| Thiamine pyrophosphatase | 0.90 | 1.20 | 1.10 |
| Glucose-6-phosphatase | 2.70 | 0.60 | 1.10 |
| Malate dehydrogenase | 1.40 | 0.08 | 0.32 |
| α-Mannosidase | 5.29 | 0.88 | 1.30 |
| Catalase | 0.84 | N.D. | 0.05 |
| N.D. = Not detected | | | |

### Example 5

### Removal of the microbody targeting signal of AT by site-directed mutagenesis of de penDE gene abolishes penicillin production

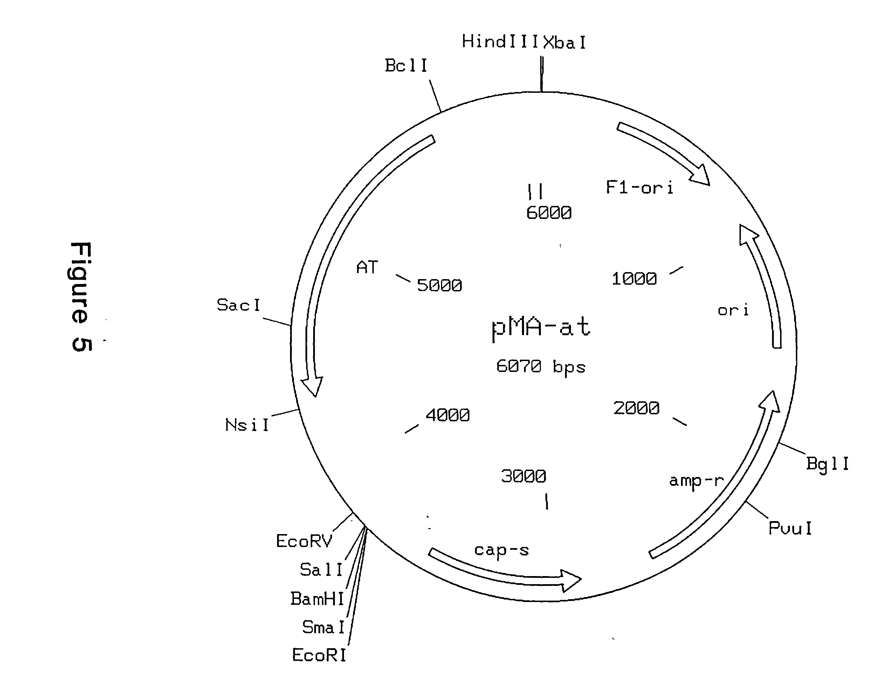

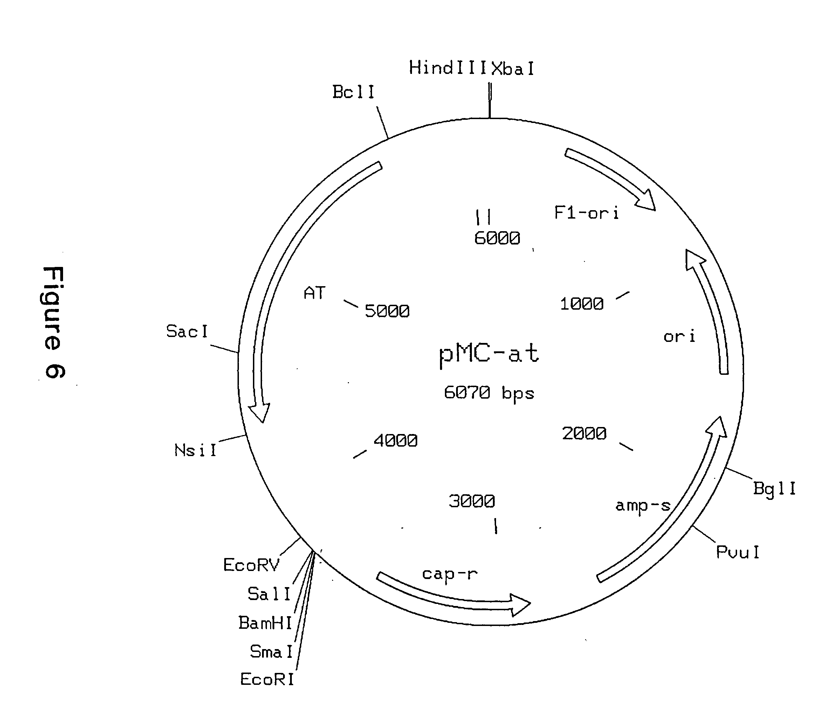

The isolation and characterization of the AT encoding penDE gene of P. chrysogenum has been described in detail (EP-A-357119; Genetics and Molecular Biology of Industrial Microorganisms, Veenstra et al., p. 262-269, 1989, American Society for Microbiology, Washington; Barredo et al., supra). The entire penDE gene is contained in the 2.3 kilo-base (kb) HindIII-SalI restriction fragment of plasmid pGJ02 (EP-A-357119). This fragment was isolated from pGJ02 by digestion with restriction enzymes HindIII and SalI and agarose gel electrophoresis, and ligated into pMA and pMC vectors, also digested with restriction enzymes HindIII and SalI. The resulting plasmids have been termed pMA-AT and pMC-AT (Figures 5 and 6, respectively).

The pMA and pMC vectors are plasmids which have been especially developed for efficient in vitro mutagenesis (Stanssens et al., Nucl. Acids Res. (1989), 17:4441-4453). E. coli strain WK6 (R. Zell and H.J. Fritz, EMBO Journal (1987), 6:1809-1815) has been used for the propagation of plasmids. Standard recDNA techniques used are adequately described in Maniatis et al. (1989, supra).

Site-directed mutagenesis of the penDE gene has been achieved by using chemically synthesized oligonucleotides (Applied Biosystems, CA, U.S.A.) containing the desired mutations. The sequence of the oligonucleotides was based upon the published nucleotide sequence of the P. chrysogenum penDE gene (Barredo et al., supra).

Oligonucleotide S,
5'-GG TCT GCG CTC AAC TGA AGG CTT TGA AGG-3', was used to change the alanine (A) encoding codon GCC in the sequence A-R-L-End into TGA, which is the authentic stopcodon of the penDE gene. Oligonucleotide D,
5'-GG TCT GCG CTC AAC TGA AGG CTC TTC-3', was used to delete the carboxy-terminal codons GCC AGG CTT, encoding A-R-L, from the penDE gene.

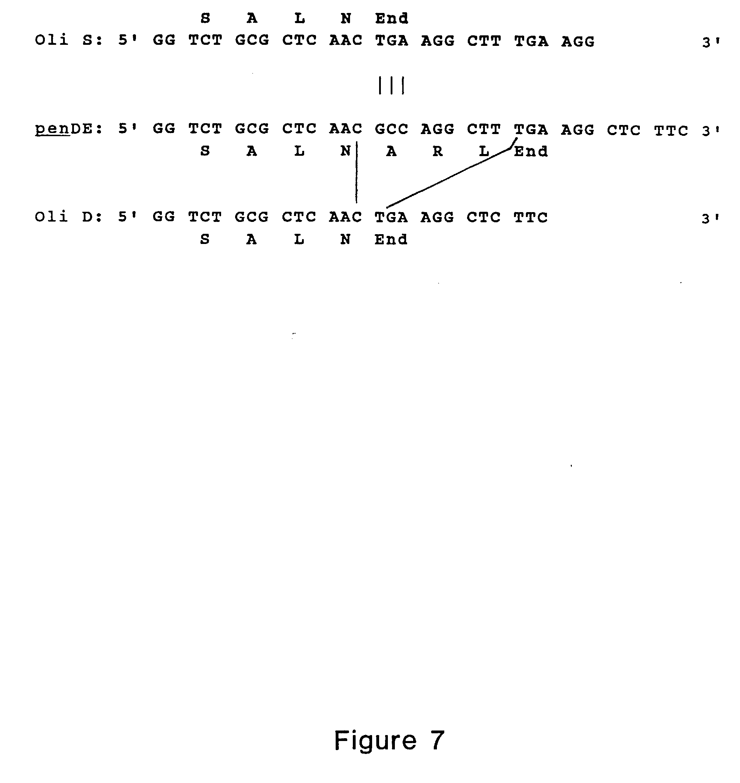

Although the mutations introduced in the penDE gene are different when using Oligonucleotide S or Oligonucleotide D, the consequences of the mutations for the structure of AT are identical. Translation of mutant penDE mRNA stops in both cases at asparagine (N) which leaves an enzyme devoid of the C-terminal ARL microbody targeting signal (Figure 7).

A gapped duplex was obtained by annealing of single-stranded (ss) DNA derived from pMA-AT with the approximately 4.8 kb SacI-NsiI restriction fragment isolated from pMC-AT. The size of the gap in the gapped duplex is approximately 320 base pairs (bp) long. Oligonucleotide S or Oligonucleotide D were subsequently annealed to this gapped duplex. The experimental conditions and procedures for the introduction of the mutations were exactly reproduced from Stanssens et al. (supra). E. coli WK6 cells containing mutant penDE genes were identified by colony hybridization by using the specific oligonucleotide used for mutagenesis as a probe. The correct introduction of desired mutations in the penDE gene was confirmed by nucleotide sequence analyses. Both Oligonucleotides S and D have been used successfully to create mutant penDE genes, which have been termed penDE-S (Oligonucleotide S) and penDE-D (Oligonucleotide D), respectively.

The penDE-S, penDE-D and penDE (as a positive control) genes have been introduced into P. chrysogenum strain npe6 as linear 2.3 kb HindIII-SalI restriction fragments, purified from pMA-AT vector sequences by co-transformation with plasmid pPS47, which contains the S. hindustanus phleomycine resistance gene as fungal selection marker (EP-A-357119), using a standard procedure for transformation of P. chrysogenum (EP-A-357119). It is obvious to those skilled in the art that other selection markers, homologous or heterologous, in the presence or absence of vector sequences, physically linked or not to the non-selectable DNA, may be used for the selection of transformants.

P. chrysogenum strain npe6 is a non-producer derivative of the Wisconsin 54-1255 strain, characterized by the absence of AT from the mycelium as was revealed by AT-antibody analyses of cell-free extracts on Western blots and by electron microscopical analyses of npe6 with AT antibodies as described in General Procedures A and B and in Example 1.

Transformants were first selected for resistance to phleomycine, purified and then tested for co-transformation of penDE genes by using a bioassay as described in EP-A-357119 and Veenstra et al., supra.

Penicillin production could be readily restored in npe6 by using the wild-type penDE gene, but restoration of penicillin production was never observed when using the penDE-S and penDE-D genes. However, the penDE-S and penDE-D genes are co-transformed and are expressed in some of the transformants as was confirmed by Western blot analyses with AT antibodies and AT activity assays on cell-free extracts. The localization of AT in these transformants was studied by electron microscopy. Specific labelling was not detected in organelles. In contrast, thin sections of penDE transformants did show specific labelling in organelles. The intensity and pattern of labelling in these transformants were identical to those observed for the parental strain Wisconsin 54-1255.

It is concluded from these experiments that targeting of AT to microbodies is mediated by the C-terminal sequence ARL. Removal of the ARL sequence from AT results in improper localization of the enzyme with a concomittant loss of penicillin production. This example demonstrates that manipulation of the microbody targeting signal is a powerful method to influence the production of secondary metabolites.

Herein the localization of penicillin biosynthetic enzymes has been investigated for the first time by a reliable electron microscopical preparation method in combination with immunogold labelling. Localization of AT, ACVS and IPNS has also been studied by cell fractionation experiments. It was surprisingly found that AT, the last enzyme in the penicillin biosynthetic pathway is located in microbodies. This is the first time that an enzyme involved in the production of secondary metabolites has been found to be located in microbodies.

Methods, such as the manipulation of the microbody targeting signal, are provided, which enable the modulation of the production of secondary metabolites such as β-lactam compounds.

Although the foregoing invention has been described in some detail by way of illustration and example for purposes of clarity and understanding, it will be readily apparent to those of ordinary skill in the art in light of the teachings of this invention that certain changes and modifications may be made thereto without departing from the spirit and scope of the appended claims.

### SEQUENCE LISTING

(1) GENERAL INFORMATION:
   (i) APPLICANT: Gist-brocades N.V., The Netherlands
   (ii) TITLE OF INVENTION: A Method of Modulating the Production of Secondary Metabolites
   (iii) NUMBER OF SEQUENCES: 5
   (iv) COMPUTER READABLE FORM: WordPerfect 5.0
   (v) CURRENT APPLICATION DATA:
      (A) APPLICATION NUMBER: 91200684.8
      (B) FILING DATE: 25 March 1991
(2) INFORMATION FOR SEQ ID NO:1:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: synthetic DNA
   (iii) HYPOTHETICAL: NO
   (iv) ANTI-SENSE: NO
   (ix) FEATURE:
      (A) NAME/KEY: PenDE Oli S
      (B) LOCATION: 1726..1737
   (xi) SEQUENCE DESCRIPTION: SEQ ID NO:1:
(2) INFORMATION FOR SEQUENCE ID NO:2:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Ser Ala Leu Asn
    1
(2) INFORMATION FOR SEQ ID NO:3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE: Penicillium chrysogenum
   (ix) FEATURE:
      (A) NAME/KEY: penDE
      (B) LOCATION: 1726..1746
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Ser Ala Leu Asn Ala Arg Leu
    1               5
(2) INFORMATION FOR SEQ ID NO:5:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: synthetic DNA
   (iii) HYPOTHETICAL: NO
   (iv) ANTI-SENSE: NO
   (ix) FEATURE:
      (A) NAME/KEY: PenDE Oli D
      (B) LOCATION: 1726..1737
   (xi) SEQUENCE DESCRIPTION: SEQ ID NO:5:

## Claims

1. A method of modulating the production of secondary metabolites in a microorganism, which comprises the use of a host microorganism, wherein the cellular localization of at least one protein, optionally derived from another microorganism, directly or indirectly involved in the production of said secondary metabolites is modulated by adding, deleting or altering one or more DNA sequences encoding one or more targeting signals for a cellular organelle in the gene(s) of one or more of said proteins.

2. Method according to claim 1 wherein the cellular organelle comprises a microbody.

3. A method of enhancing the production of secondary metabolites in a microorganism according to claim 1 or 2.

4. Method according to claim 1 to 3, comprising a co-localization of two or more enzymes, which are involved in biosynthetic routes to different secondary metabolites and which share one or more reaction steps, in microbodies.

5. Method according to any one of the preceding claims, wherein said secondary metabolites are β-lactam compounds, preferably penicillins or cephalosporins.

6. A gene encoding an enzyme involved in the production of secondary metabolites in a microorganism wherein said gene has been modified by adding, deleting or altering one or more DNA sequences encoding one or more targeting signals.

7. A DNA-construct comprising a gene according to claim 6.

8. Method according to any one of the claims 1-5, comprising the use of Penicillium chrysogenum, Aspergillus nidulans or Acremonium chrysogenum as microorganism producing said secondary metabolites.

9. Method according to any one of the claims 1-5, comprising the use of a microorganism by nature not able to produce said secondary metabolites.

10. Transformed Penicillium chrysogenum, Acremonium chrysogenum or Aspergillus nidulans, wherein the cellular localization of at least one protein, optionally derived from another microorganism, directly or indirectly involved in the production of said secondary metabolites is modulated by adding, deleting or altering one or more DNA sequences encoding one or more targeting signals in the gene(s) of one or more of said proteins.

## Patentansprüche

1. Verfahren zur Modulation der Bildung von sekundären Metaboliten in einem Mikroorganismus, umfassend die Verwendung eines Wirtsmikroorganismus, wobei die zelluläre Lokalisation mindestens eines Proteins, das gegebenenfalls von einem weiteren Mikroorganismus abgeleitet ist und direkt oder indirekt an der Bildung des sekundären Metaboliten beteiligt ist, moduliert wird, indem man eine oder mehrere DNA-Sequenzen, die für ein oder mehrere Zielsignale für eine zelluläre Organelle in dem oder den Genen von einem oder mehreren dieser Proteine kodieren, addiert, deletiert oder verändert.

2. Verfahren nach Anspruch 1, wobei die zelluläre Organelle ein Mikrokörperchen umfaßt.

3. Verfahren zur Verstärkung der Bildung von sekundären Metaboliten in einem Mikroorganismus nach Anspruch 1 oder 2.

4. Verfahren nach Anspruch 1 bis 3, umfassend eine Co-Lokalisation von zwei oder mehr Enzymen, die an biosynthetischen Wegen zu unterschiedlichen sekundären Metaboliten beteiligt sind und die eine oder mehrere Reaktionsstufen gemeinsam haben, in Mikrokörperchen.

5. Verfahren nach einem der vorstehenden Ansprüche, wobei es sich bei den sekundären Metaboliten um β-Lactamverbindungen und vorzugsweise um Penicilline oder Cephalosporine handelt.

6. Gen, das für ein Enzym kodiert, das bei der Bildung von sekundären Metaboliten in einem Mikroorganismus beteiligt ist, wobei das Gen durch Addieren, Deletieren oder Verändern von einer oder mehreren DNA-Sequenzen, die für ein oder mehrere Zielsignale kodieren, modifiziert worden ist.

7. DNA-Konstrukt, umfassend ein Gen nach Anspruch 6.

8. Verfahren nach einem der Ansprüche 1 bis 5, umfassend die Verwendung von Penicillium chrysogenum, Aspergillus nidulans oder Acremonium chrysogenum als Mikroorganismus, der die sekundären Metaboliten bildet.

9. Verfahren nach einem der Ansprüche 1 bis 5, umfassend die Verwendung eines Mikroorganismus, der von Natur aus nicht zur Bildung der sekundären Metaboliten befähigt ist.

10. Transformiertes Penicillium chrysogenum, Acremonium chrysogenum oder Aspergillus nidulans, wobei die zelluläre Lokalisation von mindestens einem Protein, das gegebenenfalls von einem weiteren Mikroorganismus abgeleitet ist und direkt oder indirekt an der Bildung des sekundären Metaboliten beteiligt ist, moduliert wird, indem man eine oder mehrere DNA-Sequenzen, die für ein oder mehrere Zielsignale in dem oder den Genen von einem oder mehreren dieser Proteine kodieren, addiert, deletiert oder verändert.

## Revendications

1. Procédé de modulation de la production de métabolites secondaires dans un micro-organisme, qui comprend l'utilisation d'un micro-organisme hôte, dans lequel la localisation cellulaire d'au moins une protéine, facultativement dérivée d'un autre micro-organisme, directement ou indirectement impliquée dans la production desdits métabolites secondaires est modulée en ajoutant, en enlevant ou en altérant une ou plusieurs séquences d'ADN codant un ou plusieurs signaux cibles pour un organite cellulaire dans le ou les gènes d'une ou plusieurs desdites protéines.

2. Procédé selon la revendication 1, dans lequel l'organite cellulaire comprend un corpuscule.

3. Procédé d'augmentation de la production des métabolites secondaires dans un micro-organisme selon la revendication 1 ou 2.

4. Procédé selon l'une quelconques des revendications 1 à 3, comprenant une colocalisation de deux ou plusieurs enzymes, qui sont impliquées dans les voies biosynthétiques vers différents métabolites secondaires et qui utilisent une ou plusieurs étapes de réaction, dans les corpuscules.

5. Procédé selon l'une quelconque des revendications précédentes, dans lequel lesdits métabolites secondaires sont des composés β-lactamiques, de préférence, des pénicillines ou des céphalosporines.

6. Gène codant une enzyme impliquée dans la production des métabolites secondaires dans un micro-organisme, dans lequel ledit gène a été modifié en ajoutant, en enlevant ou en altérant une ou plusieurs séquences d'ADN codant un ou plusieurs signaux cibles.

7. Construction d'ADN comprenant un gène selon la revendication 6.

8. Procédé selon l'une quelconque des revendications 1-5, comprenant l'utilisation de Penicillium chrysogenum, d'Aspergillus nidulans ou d'Acremonium chrysogenum comme micro-organisme produisant lesdits métabolites secondaires.

9. Procédé selon l'une quelconque des revendications 1-5, comprenant l'utilisation d'un micro-organisme par nature incapable de produire lesdits métabolites secondaires.

10. Penicillium chrysogenum, Aspergillus nidulans ou Acremonium chrysogenum transformé, dans lequel la localisation cellulaire d'au moins une protéine, facultativement dérivée d'un autre micro-organisme, directement ou indirectement impliquée dans la production desdits métabolites secondaires, est modulée en ajoutant, en enlevant ou en altérant une ou plusieurs séquences d'ADN codant un ou plusieurs signaux cibles dans le ou les gènes de l'une ou plusieurs desdites protéines.
